# EUROPÄISCHE PATENTSCHRIFT

(11) **EP 2 453 961 B1**
(45) Veröffentlichungstag und Bekanntmachung des Hinweises auf die Patenterteilung: **08.11.2017**
(21) Anmeldenummer: 10724801.5
(22) Anmeldetag: 07.06.2010
(51) Int. Cl.: A61M 11/02, A61M 11/06, A61M 15/00, B05B 1/00, B05B 11/00, B05B 15/00, A61B 17/3203

(54) **HOCHDRUCKKAMMER**
HIGH-PRESSURE CHAMBER
CHAMBRE À HAUTE PRESSION

(30) Priorität: 13.07.2009 EP 09165311
(43) Veröffentlichungstag der Anmeldung: 23.05.2012
(73) Patentinhaber: Boehringer Ingelheim International GmbH, 55216 Ingelheim am Rhein (DE)
(72) Erfinder: HAUSMANN, Matthias, 55216 Ingelheim am Rhein (DE); WABNITZ, Thomas, 55216 Ingelheim am Rhein (DE)
(74) Vertreter: Simon, Elke Anna Maria
(86) Internationale Anmeldenummer: PCT/EP2010/057937
(87) Internationale Veröffentlichungsnummer: WO 2011/006711

(56) Entgegenhaltungen:
- WO-A-94/07607
- WO-A-2006/126014
- DE-A1- 1 653 651
- DE-A1-102004 031 673
- US-A1- 2002 176 788
- FUH-KUO CHEN ET AL: "A study of forming pressure in the tube-hydroforming process" JOURNAL OF MATERIALS PROCESSING TECHNOLOGY, ELSEVIER, NL, Bd. 192-193, 1. April 2007 (2007-04-01), Seiten 404-409, XP007910052 ISSN: 0924-0136

## Beschreibung

Die vorliegende Erfindung betrifft eine Hochdruckkammer, die aus mehreren Einzelteilen zusammengesetzt ist und mit Flüssigkeit betrieben wird.

Gegenstand der vorliegenden Erfindung sind Hochdruckkammern für medizintechnische Anwendungen, wie beispielsweise bei der Zerstäubung von flüssigen Arzneimittelformulierungen zur Aufnahme in der Lunge eines Patienten oder bei der Injizierung in den Körper eines Patienten.

Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen. Insbesondere bezieht er sich auf den Aggregatzustand des Inhalts der Hochdruckkammer während der Benutzung.

Es ist bekannt, dass bei den meisten medizinischen Verfahren, bei denen Flüssigkeiten in den Körper eines Patienten appliziert oder innerhalb von medizintechnischen Geräten geleitet werden, mit sehr niedrigen Drücken gearbeitet wird, beispielsweise beim Anlegen von Infusionen oder bei medizinischen Verfahren, in denen Blut oder andere physiologische Flüssigkeiten zirkuliert werden. Auch übliche Spendersysteme für Kosmetika oder medizinische Druckgaszerstäuber arbeiten in der Regel unterhalb von 7 bar Flüssigkeitsdruck, ganz selten bis zu 20 bar. Hochdruckpumpsysteme andererseits sind zwar generell von diversen industriellen Anwendungen her bekannt, aber kaum für den Einsatz in medizintechnischen Geräten geeignet, da sie häufig aus Materialien gefertigt werden, die nicht mit den speziellen medizinischen Wirkstoffen verträglich sind oder gar für die Patienten toxische Substanzen freisetzen können. Häufig wird bei medizintechnischen Bauteilen deren Sterilisierbarkeit gefordert. Bauteile für medizinische Geräte sind aufgrund der notwendigen Hygieneforderungen meist für kurze Anwendungszeiträume oder gar Einmalanwendungen ausgelegt, so dass in diesem Bereich die Forderung der Massenfertigbarkeit deutlich verstärkt ist. Darüber hinaus sind industrielle Pumpsysteme häufig mechanisch komplex aufgebaut, so dass sie schwerlich insbesondere auf die Größe von medizinischen Handgeräten verkleinert werden können.

Bei der Therapie von Lungenerkrankungen ist der Einsatz von portablen Handgeräten mittlerweile unersätzlich. Mit ihrer Hilfe können Therapien täglich auch fern der Praxis des behandelnden Arztes durchgeführt werden, und so kann ein Patient ein für ihn wichtiges Notfallmedikament zum Einatmen immer greifbar dabei haben.

Bei der Zerstäubung einer flüssigen Arzneimittelformulierung soll eine genau dosierte Menge an Wirkstoff in ein Aerosol zur Inhalation überführt werden. Das Aerosol soll hierbei eine kleine mittlere Tröpfchengröße bei einer schmalen Tröpfchengrößenverteilung haben. Um dieses mit Hilfe von Düsen-Pumpenanordnungen ohne den Gebrauch von Treibmitteln zu erreichen, sind in den zugehörigen Pumpenkammern Drücke von 100 bis 1200 bar bei hohen Forderungen an die Abdichtung des Systems notwendig.

Unter dem Begriff "Arzneimittelformulierung" sind bei der vorliegenden Erfindung über Medikamente hinaus auch Therapeutica oder dergleichen, insbesondere also jede Art von Mitteln zur Inhalation oder sonstigen Anwendung zu verstehen.
Die vorliegende Erfindung ist jedoch nicht auf medizinische Zerstäubung beschränkt, sondern kann Gebiets-übergreifend für die Abgabe jeglicher Flüssigkeiten unter Druck wie z.B. insbesondere bei der Abgabe von abgemessenen Flüssigkeitsmengen in Injektoren, Spraysystemen und anderen Spendersystemen sowie bei Systemen, in denen Flüssigkeitsstrahlen mit hohem Druck (z.B. in Schneidsystemen) verwendet werden, auch wenn die nachfolgende Beschreibung primär auf medizinische Anwendungen und die bevorzugte Zerstäubung einer Arzneimittelformulierung zur Inhalation gerichtet ist. Darüber hinaus können derartige Hochdruckkammer und die hiermit verbundenen Fertigungstechniken in ganz anderen technischen Gebieten wie z.B. der Motorenindustrie verwendet werden, obwohl sich diese Erfindung primär auf solche Pumpsituationen bezieht, bei denen eine besonders reine Handhabung der jeweiligen Flüssigkeit erforderlich ist, wie es z.B. insbesondere in der Medizintechnik, pharmazeutischen Industrie oder Lebensmitteltechnik der Fall ist.

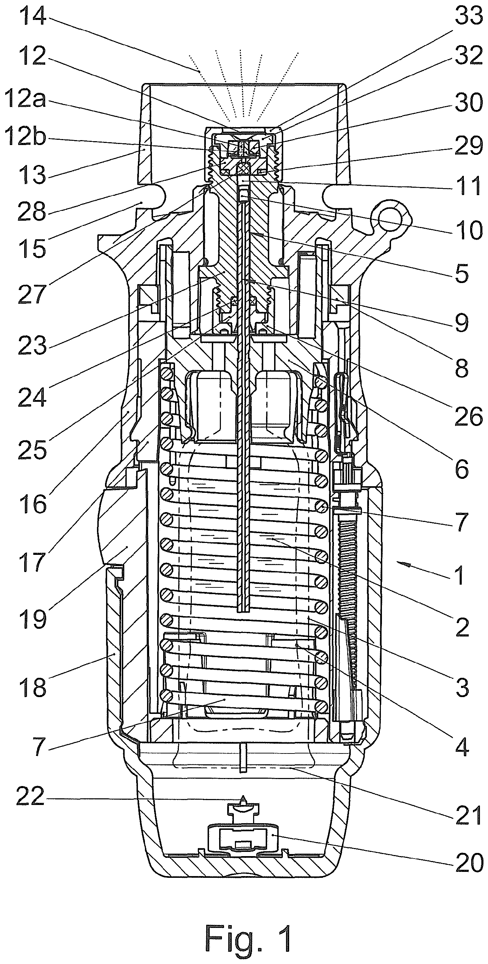

In der WO91/14468 A1 und in der WO97/12687 A1 sind Zerstäuber bzw. miniaturisierte Hochdruckzerstäuber beschrieben. Diese weisen als Reservoir für eine zu zerstäubende Arzneimittelzubereitung einen einsetzbaren, starren Behälter mit Innenbeutel und einen manuell betätigten Druckerzeuger mit einer Antriebsfeder zum Fördern und Zerstäuben der Arzneimittelzubereitung auf. Ein solcher Behälter, wie in der WO96/06011 A1 und WO00/49988 A2 offenbart, nimmt ein Volumen von ca. 2 bis 10 ml auf. Ein zu den oben genannten Zerstäubern alternativer Zerstäuber ist mittlerweile aus dem Stand der Technik bekannt und wie als Beispiel in Fig. 1 dargestellt und weiter unten im Detail erläutert. In diesem Zerstäuber ist eine mehrteilige Pumpenkammer eingesetzt. Die festigkeitsbestimmenden Komponenten dieser weitgehend kreiszylindrischen Pumpenkammer sind ein Führungsrohr für den längs verschiebbar geführten Pumpenkolben, Reservoir-seitig ein Stützring, der durch ein angeschraubtes kappenförmiges Halteelement fixiert wird und an dem anderen Ende der Pumpenkammer ein von einem ähnlichen angeschraubten kappenförmigen Halteelement fixierter Düsenhalter. Details möglicher Mikrostrukturen für die vom Düsenhalter gehaltene Austragsdüse werden in den Schriften WO94/07607, WO99/16530 und WO2005/000476A1 offenbart.

Die für die Pumpenkammer eingesetzten Bauteile eingesetzten Bauteile unterliegen besonderen Forderungen an die Festigkeit des Materials. Sie können häufig nicht aus den sonst für massengefertigte Bauteile in der Medizintechnik üblichen, vergleichsweise preiswerten Kunststoffen hergestellt werden. Bei den beschriebenen Halteelementen handelt es sich typischerweise um auf Drehmaschinen gefertigte Bauteile aus Metall.

US2002/0176788 zeigt unter anderem einen Hochdruckpumpenkörper, dessen Wand aus einem dünnwandigen Rohr besteht und bei dem die festigkeitsbestimmenden Komponenten nicht zusammengeschraubt sind, sondern über eine Dichtsicken-Crimpverbindung mit Anschlusselementen verbunden sind. Ein Auslassventil mit Dichtelementen ist in das dünnwandige Rohr eingeschoben. Das Rohr ist an der Stelle einer geeigneten Aussparung in der Ventileinheit zu dieser hin formschlüssig eingedrückt. Bei Erreichen des vorgesehenen Hochdrucks im Hochdruckpumpenkörper öffnet sich das entsprechende Ventil und lässt die Flüssigkeit kontinuierlich zum Pumpenauslass fließen.

Der vorliegenden Erfindung liegt die Aufgabe zugrunde, eine Hochdruckkammer mit integrierter Auslassdüse insbesondere für medizinische Zerstäuber- oder Injektorsysteme anzugeben, die für die industrielle Fertigung geeignet ist. Solche Systeme geben in kurzen Pulsen eine dosierte Flüssigkeitsmenge aus. Hierbei wird die Flüssigkeit druckfrei angesaugt und innerhalb kurzer Zeit in der Hochdruckkammer auf einen Spitzendruck gebracht, bei dem die Flüssigkeit über eine Düse (vorzugsweise direkt) ausgegeben wird. Die Dichtigkeitsforderungen an ein solches gepulste System haben im Vergleich zum kontinuierlichen Pumpen nicht nur statische, sondern zusätzlich dynamische Aspekte, so dass besondere Forderungen an die Verbindungtechnik - insbesondere zwischen Hochdruckkammer und Auslassdüse - gestellt werden.

Unter dem Begriff Hochdruckkammer ist hierbei eine innen weitgehend kreiszylindrische Kammer zu verstehen, in welcher eine Flüssigkeit durch Kolbenvortrieb unter Druck gesetzt und ausgestoßen wird.

Diese Aufgabe wird erfindungsgemäß gelöst durch eine Hochdruckkammer gemäß Anspruch 1 und durch ein Verfahren zur Herstellung einer Hochdruckkammer gemäß Anspruch 15. Die erfindungsgemäße Hochdruckkammer hat die Form einer aus mehreren Bauteilen zusammengesetzten Kolbenpumpenkammer und weist ein Einlassventil und eine Auslassdüse auf. In der Hochdruckkammer wird eine Flüssigkeit unter Hochdruck gesetzt und durch einen axial verschiebbaren Kolben durch die Auslassdüse ausgestoßen. Mindestens ein Bauteil besteht aus einem umformbaren und /oder crimpfähigen und/oder bördelbaren und/oder verquetschbaren Metallbauteil. Das mindestens eine Bauteil ist mit mindestens einem anderen Bauteil der Kammer formschlüssig, kraftschlüssig und unlösbar verbunden. Vorteilhafte Weiterbildungen werden im Folgenden und im Detail anhand der Figuren beschrieben.

Ein Merkmal der vorliegenden Erfindung ist, die Geometrie von festigkeitsbestimmenden Bauteilen bereits z.B. unter Ausnutzung spezieller im weiteren beschriebenen Fertigungsverfahren so zu gestalten, dass einige Bauteile Elemente enthalten, mit denen diese Bauteile mit anderen Bauteilen verbunden werden. Diese Verbindungselemente können vorzugsweise als Ärmchen, Stege, Zackenkränze (ähnlich wie bei Kronkorken) oder andersartig gestaltet sein. Die Verbindungselemente werden beim formschlüssigen Fügen zweier Bauteile verformt oder verbogen, vorzugsweise gecrimpt. Vorzugsweise wird ein crimpfähiges Bauteilmaterial gewählt, das bei den vorliegenden Geometrien im Verformungsbereich duktil ist und hinreichend verformbar ist. Bevorzugt ist ein solches Material, das sich nach einer mechanischen Verformung im verformten Bereich verfestigt.

Die Crimpverbindung von Bauteilen ermöglicht die schnellere Montage der Bauteile im Vergleich z.B. zu Schraubverbindungen. Weiter sind Crimpverbindungen bezüglich der Lagetoleranzen weniger fehleranfällig als Schraubverbindungen. Crimpverbindungen sind sehr robust und können mit Innendrücke von bis zu 5000 bar belastet werden.
Die festigkeitsbestimmenden Bauteile der Hochdruckkammer werden bevorzugt am Ende des Montageprozesses durch Crimpen miteinander verbunden. Hierdurch entsteht eine Bauteileinheit, in der alle funktionalen Komponenten des Systems - wie z.B. Dichtelemente, Filterelemente und Düsen - eingeschlossen sind.
Die hier beschriebene Hochdruckkammer eignet sich aufgrund ihrer kompakten Bauweise gut zum Einbauen in medizinische Handgeräte.

Ein weiteres Merkmal der vorliegenden Erfindung ist, dass im Metall-Spritzguss-Verfahren (MIM-Technologie) gefertigte Metallbauteile als festigkeitsbestimmende Bauteile in Hochdruckkammern eingesetzt werden. Die Abkürzung MIM bedeutet "metal injection moulding". Das MIM-Verfahren ist ein Metall-Spritzguss-Prozess, bei dem ein metallisches Pulver (hier sind alle bekannten pulverförmigen Metalle und Legierungen verwendbar) mit Bindematerial, z.B. mit Polyolefin, im Spritzgussprozess zum gewünschten Bauteil abgeformt und anschließend in mehreren Verfahrensschritten bei unterschiedlichen Temperaturen behandelt wird. Hierbei wird dem Bauteil zuerst das Bindematerial entzogen, und anschließend wird das Bauteil durch Sintern gehärtet. In der Regel werden im MIM-Verfahren Metallpulver mit Korngrößen kleiner als 30 Mikrometer und einem Median der Korngrößenverteilung von 6 bis 7 Mikrometern eingesetzt. Mit diesem Verfahren können Bauteile massenweise und wirtschaftlich hergestellt werden. Diese Bauteile sind ohne Nachbearbeitung, z.B. auf Drehmaschinen, zu verwenden. Die Geometrie der Bauteile aus Sintermetall kann gegenüber konventionell hergestellten Bauteilen aus Metall in vielfältiger Weise variiert werden - so ist die Geometrie der Bauteile aus Sintermetall beispielsweise nicht auf rotationssymmetrische Formen beschränkt ist. Zusätzlich können durch die Wahl des Ausgangspulvers besonders korrosionsbeständige Materialien für die Bauteile eingesetzt werden, wodurch die Robustheit des Systems gesteigert wird.

Ein weiteres Merkmal der vorliegenden Erfindung ist, Kunststoffelemente oder Kunststoffbeschichtungen direkt an festigkeitsgebende Komponenten der Hochdruckkammer (vorzugsweise vor dem Umformen oder dem Zusammenfügen) anzubringen. Dies kann über die Kombination von Herstellungsverfahren realisiert werden, indem z.B. ein Bauteil aus Sintermetall als Einlegeteil bei einem Kunststoff-Spritzguss-Prozess oder einem Beschichtungsverfahren verwendet wird (im Falle des Kunststoff-Spritzguss-Prozesses wird ein weiter prozessierte Einlegeteil unabhängig vom Grad der Oberflächenbedeckung mit Kunststoff als "umspritztes Teil" bezeichnet, um aber andere Beschichtungsprozesse im Folgenden sprachlich mit zu erfassen wird hier die Bezeichnung "Grundkörper" gewählt). Desgleichen können Metallbauteile aus anderen Fertigungs- oder Strukturierungsprozessen verwendet werden. Auf diese Weise kann man vorteilhaft verschiedene Forderungen an Ausführung und Funktion in einem einzigen Bauteil erfüllen.
Die umspritzten Metallbauteile oder Grundkörper bieten die für Hochdruckanwendungen nötige Steifigkeit. Mit dem Beschichten mit Kunststoff oder dem Anbinden von Kunststoffelementen können je nach Position und Flüssigkeitskontakt und je nach Materialwahl sowohl elastische Dichtelemente als auch die pharmazeutische Verträglichkeit und der Korrosionsschutz der Bauteile realisiert werden. Bevorzugt wird hier das Beschichten - z.B. mit Polypropylen - aller Bauteiloberflächen, die in direktem Kontakt mit der Flüssigkeit stehen, d.h. insbesondere der Oberflächen im Innenraum der Hochdruckkammer. Hiermit wird bei empfindlichen Wirkstoffen die Verträglichkeit zwischen Bauteil und Wirkstoff erreicht. Anstelle von Polypropylen ist je nach gewünschtem Effekt das Anspritzen von Elementen aus oder das Umspritzen des Metallbauteils mit einer Vielzahl von handelsüblichen Kunststoffen aus der Gruppe der Thermoplaste, Duroplaste oder Elastomere möglich.

Die Auskleidung des Innenraums eines Bauteils aus Sintermetall mit Kunststoff hat bei seinem Einsatz als Komponente einer gepulst betriebenen Hochdruckkammer den weiteren Vorteil, dass je nach Wahl des Metallpulvers für den Spritzgussprozess im gesinterten Bauteil zurückgebliebene oberflächige Poren verschlossen werden und die Oberfläche geglättet wird. Auf diese Art und Weise kann selbst bei groben Pulvern vermieden werden, dass das Innenvolumen der Hochdruckkammer und damit das Totvolumen durch Poren in der Oberfläche des Sintermetalls erhöht wird. Je geringer das Totvolumen in einem Pumpensystem ist, umso kürzer ist die Anlaufphase des Systems (bei der Beschreibung eines Zerstäubers wird diese Anlaufphase später als Primen bezeichnet) und umso genauer ist die Dosierung der Flüssigkeit bei gepulsten Systemen.

Die einzelnen Merkmale der vorliegenden Erfindung können größtenteils unabhängig voneinander genutzt und/oder weitgehend beliebig miteinander kombiniert werden.
Weitere Merkmale und Eigenschaften der vorliegenden Erfindung werden in der folgenden Beschreibung und anhand der Zeichnungen weiter erläutert. Die Zeichnungen zeigen in
- Fig. 1: einen schematischen Schnitt eines bekannten Zerstäubers im ungespannten Zustand,
- Fig. 2: einen schematischen, um 90° gegenüber Fig.1 gedrehten Schnitt des Zerstäubers aus Fig. 1 im gespannten Zustand,
- Fig. 3: einen schematischen Schnitt durch die erfindungsgemäße Hochdruckkammer zum Einsatz in einem Zerstäuber
- Fig. 4a: den Grundkörper eines Bauteils (Düsenhalter) der Hochdruckkammer aus Fig. 3 vor Anbindung eines zweiten Materialbereichs und vor Montage
- Fig. 4b: den gleichen Grundkörper aus Fig. 4a aus einer anderen Perspektive
- Fig. 5: das fertige Bauteil aus Fig. 4 einschließlich des angebundenen zweiten Materialbereichs vor Befestigung an die Hochdruckkammer

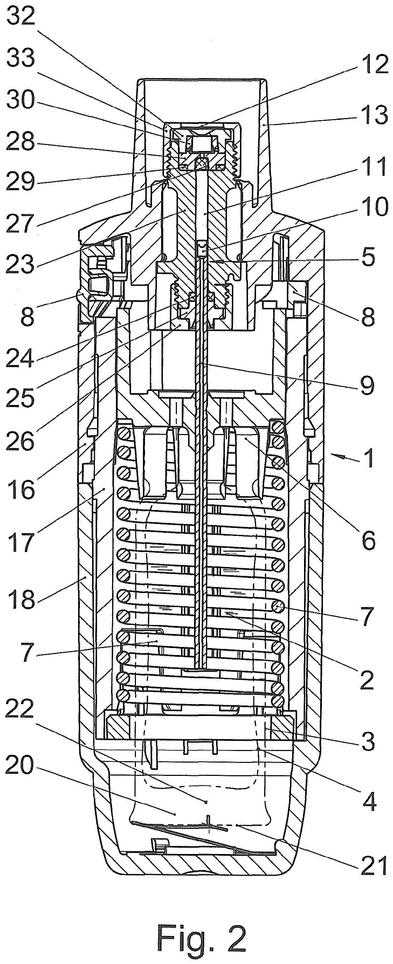

Fig. 1 und 2 zeigen in einer schematischen Darstellung einen bekannten handbetätigten Zerstäuber (1) zur Zerstäubung einer Flüssigkeit (2), in dem die Druckkammer (11) durch die vorschlagsgemäße Hochdruckkammer ersetzt werden kann. Bei dem Zerstäuber aus Fig. 1 und (2) handelt es sich um einen Dosierzerstäuber, der in jedem Betätigungszyklus eine vorgegebene Flüssigkeitsdosis ausgibt. Beim Betrieb des Zerstäubers wird unterschieden zwischen dem ungespannten Zustand mit unbefülltem Dosiervolumen in der Druckkammer (11) (Fig. 1) und dem gespannten Zustand mit befüllter Druckkammer (11) (Fig. 2). Die Druckkammer (11) entspricht hierbei in ihrer Funktion einer Pumpenkammer.
Die festigkeitsbestimmenden Komponenten dieser Druckkammer sind ein im Innern im wesentlichen zylindrisches Zentralteil (23) - beispielsweise aus einem festen Kunststoff wie vorzugsweise PEEK -, ein mit diesem Zentralteil (23) stromaufwärts mittels Überwurfmutter (26) verschraubter Stützring (25) und ein stromabwärts mit dem Zentralteil (23) mittels Überwurfinuttern (33) verschraubter Düsenhalter (32). Diese festigkeitsbestimmenden Komponenten schließen im montierten Zustand eine Reihe von funktionstragenden Komponenten - diverse Dichtungen, Filter und Düse - mit ein, die später erläutert werden.

Fig. 1 und 2 zeigen den Zerstäuber (1) mit einem Behälter (3) mit der Flüssigkeit (2). Der Zerstäuber (1) ist als tragbares Handgerät ausgebildet und arbeitet ohne Treibgas. Vorzugsweise kann er als medizinischer Zerstäuber zur Inhalation von flüssigen Arzneimittelformulierungen verwendet werden.

Bei Zerstäubung der Flüssigkeit (2), wird ein Aerosol (14) (Fig. 1) gebildet, das im Falle eines medizinischen Zerstäubers vorzugsweise lungengängig ist und von einem nicht dargestellten Benutzer eingeatmet und gegebenenfalls mehrmals täglich inhaliert werden kann.

Der Behälter (3) mit der Flüssigkeit (2) ist nach Öffnen des Zerstäubers (1) von unten in diesen einsetzbaren und/oder wechselbar. Der Behälter (3) bildet ein Reservoir für die zu zerstäubende Flüssigkeit (2). Vorzugsweise enthält der Behälter (3) eine ausreichende Menge an Flüssigkeit (2) für mehrere Dosen der Flüssigkeit (2), beispielsweise bis zu 200 Dosiereinheiten (Dosen) für bis zu 200 Zerstäubungen oder Anwendungen.
Der Zerstäuber (1) weist ferner eine Fördereinrichtung, insbesondere einen Druckerzeuger (5), zur Förderung und Zerstäubung der Flüssigkeit (2), jeweils in einer vorbestimmten, ggf. einstellbaren Dosiermenge auf.

Der Zerstäuber (1) weist insbesondere eine Halterung (6) für den Behälter (3), eine zugeordnete, nur teilweise dargestellte Antriebsfeder (7) vorzugsweise mit einem zum Entsperren manuell betätigbaren Sperrelement (8), einen als Kapillare ausgebildeten Hohlkolben (9) mit einem Ventil, insbesondere Rückschlagventil (10), eine Druckkammer (11) und eine Austragsdüse (12) im Bereich eines Mundstücks (13) auf. Das Rückschlagventil (10) umfasst vorzugsweise einen Ventilkörper, der in einer entsprechenden endseitigen Ausnehmung des Hohlkolbens (9) axial begrenzt beweglich ist. Insbesondere ist der Ventilkörper seitlich und/ oder stirnseitig (zur Druckkammer (11) hin) mit einer Ausnehmung, Nut oder dergleichen versehen, so dass er bei geöffnetem Rückschlagventil (10) von der Flüssigkeit (2) umströmbar ist, auch wenn der Ventilkörper zur Druckkammer hin an einem axialen Anschlag des Hohlkolbens (9) bei geöffnetem Rückschlagventil (10) anliegt.

Der Behälter (3) wird über die Halterung 6, insbesondere klemmend oder rastend, so in dem Zerstäuber (1) fixiert, dass das untere Ende des Hohlkolbens (9) in den Behälter (3) eintaucht. Die Halterung (6) kann dabei derart ausgebildet sein, dass der Behälter (3) ausgetauscht werden kann.

Beim axialen Spannen der Antriebsfeder (7) wird die Halterung (6) mit dem Behälter (3) und dem Hohlkolben (9) nach unten bewegt und eine Dosis der Flüssigkeit (2) aus dem Behälter (3) in die Druckkammer (11) des Druckerzeugers (5) am Rückschlagventil (10) vorbei gesaugt.

Beim anschließenden Entspannen nach Betätigen des Sperrelements (8) wird die Flüssigkeitsdosis in der Druckkammer (11) unter Druck gesetzt, indem der Hohlkolben (9) bei nun geschlossenem Rückschlagventil (10) durch Entspannen der Antriebsfeder (7) wieder nach oben bewegt wird und dabei als Druckkolben wirkt. Dieser Druck treibt die Flüssigkeit in der Druckkammer (11) durch die Austragsdüse (12) aus, wobei sie in das vorzugsweise lungengängige Aerosol (14) zerstäubt wird, wie in Fig. 1 angedeutet. Hierbei treten typischerweise Druckspitzen von mehreren 100 bar, teilweise bis zu 1200 bar im System auf. Die Querschnittsfläche der Druckkammer und der Hubweg des Druckkolbens bestimmen das Volumen der durch die Austragsdüse zerstäubten Flüssigkeit. Hierbei ist das Innenvolumen der Hochdruckkammer bevorzugt nur geringfügig größer als das vom Druckstempel verdrängte Volumen. Bevorzugt werden Hochdruckkammern mit bis zu 250 Mikroliter Innenvolumen, insbesondere mit Volumina zwischen 10 und 100 Mikrolitern.

Der nicht dargestellte Benutzer kann das Aerosol (14) inhalieren, wobei vorzugsweise Zuluft über mindestens eine Zuluftöffnung (15) in das Mundstück (13) angesaugt wird.

Während des Zerstäubungsvorgangs wird der Behälter (3) von der Antriebsfeder (7) wieder in seine Ausgangslage zurückbewegt. Er führt also eine Hubbewegung während des Spannvorgangs und während des Zerstäubungsvorgangs aus.

Der Zerstäuber (1) weist ein Gehäuseoberteil (16) und ein demgegenüber drehbares Gehäuseinnenteil (17) (Fig. 2) auf, wobei an dem Gehäuseinnenteil (17) ein manuell betätigbares oder drehbares Gehäuseunterteil (18) vorzugsweise mittels eines Halteelementes (19) lösbar befestigt, insbesondere aufgesteckt, ist. Zum Einsetzen und/oder Auswechseln des Behälters (3) ist das Gehäuseunterteil (18) vom Gehäuseinnenteil (17) lösbar, so dass der Behälter in das Gehäuseinnenteil (17) axial eingeschoben werden kann. Das Gehäuseunterteil (18) ist vorzugsweise kappenartig ausgebildet und greift um oder über den unteren freien Endbereich des Behälters (3).

Das Gehäuseunterteil (18) kann gegen das Gehäuseoberteil (16) gedreht werden, wobei es das Gehäuseinnenteil (17) mitnimmt. Dadurch wird die Antriebsfeder (7) über ein nicht dargestelltes, auf die Halterung (6) wirkendes Getriebe in axialer Richtung gespannt. Mit dem Spannen wird der Behälter (3) axial nach unten und mit seinem Endbereich weiter in das Gehäuseunterteil (18) zu dessen stirnseitigem Ende hin bewegt, bis der Behälter (3) die in Fig. 2 dargestellte Endlage einnimmt. In diesem Zustand ist die Antriebsfeder (7) gespannt.

Beim erstmaligen Spannen wird der Behälter (3) bodenseitig angestochen und dadurch belüftet. Eine axial wirkende, im Gehäuseunterteil (18) angeordnete Feder (20) stößt hierbei am Behälterboden (21) an, wobei das an der Feder (20) angebrachte Anstechelement (22) den Behälter (3) oder eine bodenseitige Versiegelung bei der erstmaligen Anlage ansticht. Hierbei wird lediglich die Außenhülle des Behälters (3) geöffnet, der innere Beutel (4) mit der Flüssigkeit (2) wird nicht angestochen. Das Innenvolumen des Beutels (4) wird mittels des Hohlkolbens 9 geöffnet, der beim Einsetzen des Behälters (4) in das Gehäuseinnenteil (17) eine kopfseitige Versiegelung des Behälters (3) durchsticht und anschließend durch ein kopfseitiges Septum des Behälters hindurch in das Innere des Beutels eingeführt wird. So wird der Beutel (4) im Behälter (3) über den Hohlkolben (9) mit der Druckkammer (11) fluidisch verbunden. Vor der ersten Benutzung nach dem fluidischen Anschließen des Beutels (4) wird der Zerstäubers (1) mehrmals gespannt und ausgelöst. Durch diesen als "Primen" bezeichneten Vorgang wird die im Hohlkolben (9) und im Druckerzeuger (5) bis zur Austragsdüse (12) vorhandene Luft von der Flüssigkeit verdrängt und der Zerstäuber (1) ist zur bestimmungsgemäßen Benutzung bereit. Beim folgenden Entnehmen von Flüssigkeit aus Beutel (4) über den Hohlkolben (9) kollabiert der flexible Beutel (4). Zum Druckausgleich im Behälter (3) kann Umgebungsluft über die Belüftungsöffnung in den Behälter (3) nachströmen, so dass immer die gleichen Druckbedingungen beim Fördern der Flüssigkeit (2) vorliegen.

Der Druckerzeuger (5) weist ein rohrförmiges Zentralteil (23) auf, das mit einer Längsbohrung versehen ist, welche die Druckkammer (11) bildet. Der Hohlkolben (9) ragt in die Druckkammer (11) hinein. Er ist mit der Dichtung (24) abgedichtet, die von einem Stützring (25) und einer Überwurfmutter (26) in einer entsprechenden Ausnehmung am behälterseitigen Ende des Zentralteils (23) gehalten wird. Der Hohlkolben (9) erstreckt sich im montierten Zustand durch die Dichtung (24) hindurch und wird durch diese außenseitig bzw. radial abgedichtet.

Am auslassseitigen Ende des Zentralteils (23) ist die Austragsdüse (12) angebracht. Zwischen der Austragsdüse (12) und der Druckkammer (11) ist vorzugsweise ein Vorfilter (27) angeordnet, der vorzugsweise aus einem gegenüber der Flüssigkeit (2) chemisch verträglichen Kunststoff wie Polyethylen oder Polypropylen hergestellt ist.

Mit dem Vorfilter (27) werden Partikel zurückgehalten, welche die nachgeordnete Austragsdüse (12) oder einen Feinfilter verstopfen oder verlegen können. Die Filterschwelle liegt vorzugsweise im Bereich von etwa 10 Mikrometer. Größere Partikel werden aus der durchströmenden Flüssigkeit (2) vom Vorfilter (27) zurückgehalten.

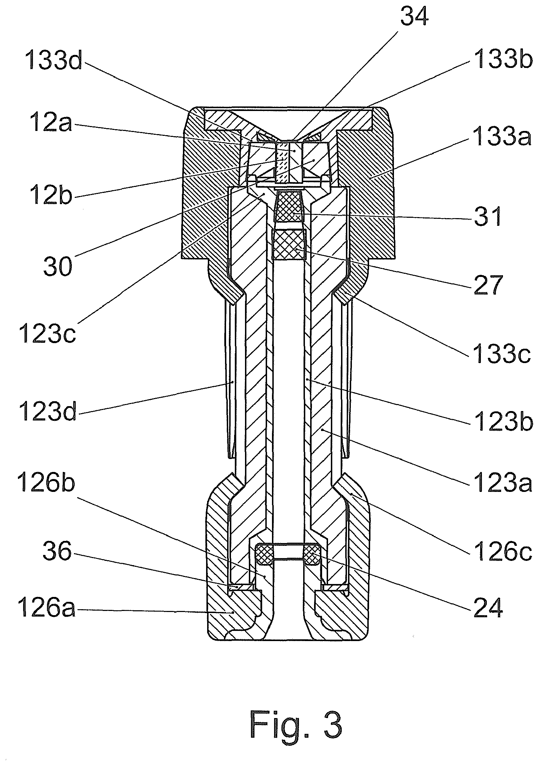

In Fig. 1 und Fig. 2 wird der Vorfilter (27) von einem Filterhalter (28) unmittelbar am auslassseitigen Ende der Druckkammer (11) - beispielsweise gepresst - gehalten, wobei der Filterhalter (28) mittels einer Dichtung (29), insbesondere eines O-Rings, gegenüber dem Zentralteil (23) abgedichtet wird. Jedoch sind andere konstruktive Lösungen möglich, wie beispielsweise ein weiteres Ausführungsbeispiel (siehe später auch Fig. 3) zeigen wird.

An den Vorfilter (27) oder den Filterhalter (28) schließt sich vorzugsweise unmittelbar ein Feinstfilter und die Austragsdüse (12) an. Besonders bevorzugt wird die zweistufige Filterung der Flüssigkeit über Vorfilter (27) und Feinstfilter vor dem Zerstäuben. Insbesondere bilden Feinstfilter und Austragsdüse (12) ein einziges Bauteil. Mittels des Feinstfilters werden Partikel herausgefiltert, welche die sehr feinen Auslasskanäle der Austragsdüse (12) verstopfen oder verlegen könnten. Die Filterschwelle liegt insbesondere bei 2 bis 5 Mikrometer.

Die Austragsdüse (12) wird vorzugsweise von einer Düsendichtung (30) aufgenommen und radial gehalten. Abgabeseitig schließt sich der Düsenhalter (32) an. Zum Befestigen am Zentralteil (23) ist die Überwurfmutter (33) vorgesehen. Mit dem Düsenhalter (32) werden die Austragsdüse (12) und die Düsendichtung (30) gehalten.
Die Austragsdüse (12) ist aus zwei fest miteinander verbundenen Teilen, den Platten (12a) und (12b) aufgebaut, die aus Glas und/oder Silizium bestehen. Eine Platte ist mikrostrukturiert und enthält auf einer Flachseite einen Strömungsbereich, der den Düseneinlass mit dem Düsenauslass verbindet. Die beiden Platten (12a) und (12b) sind vorzugsweise durch Bonden fest miteinander verbunden. Der Strömungsbereich mit dem stromaufwärts gelegenen Düseneinlass und den stromabwärts gelegenen Düsenauslass ist zwischen beiden Platten eingeschlossen. Die zwischen den Platten liegenden Mikrostrukturen bilden im Strömungsbereich entlang der Strömungsrichtung zunächst eine Feinstfilterstruktur und anschließend den Düsenkanal mit Düsenauslass. Diese Feinstfilterstruktur wird beispielsweise aus engen Durchlässen durch Wandungen und/oder Erhebungen mit bestimmter Flächendichte gebildet.
Das Volumen der sich an die Druckkammer (11) anschließenden fluidischen Verbindung zur Austragsdüse (12) und das Volumen der Austragsdüse (12) selbst sind möglichst klein, um ein geringes Totvolumen zu erreichen. Der Strömungswiderstand durch die Austragsdüse (12) ist wesentlich höher als der Strömungswiderstand der durch den Hohlkolben beim Spannhub in die Druckkammer (11) einströmenden Flüssigkeit (2), so dass die Funktion des Zerstäubers ohne ein auslassseitiges Ventil im Düsenbereich gegeben ist.

Der Zerstäuber arbeitet mit einem Federdruck von 50 bis 600 bar, bevorzugt 100 bis 500 bar. Bei jeder Betätigung des Zerstäubers wird ein Flüssigkeitsvolumen von 10 bis 50 Mikrolitern ausgebracht. Die Flüssigkeit wird in ein Aerosol überführt, dessen Tröpfchen einen aerodynamischen Durchmesser von bis zu 20 Mikrometer, bevorzugt von 3 bis 10 Mikrometer, haben. Die zugehörigen Düsen erzeugen dabei eine Strahlfächerung von 20° bis 160°, bevorzugt von 80° bis 100°. Derartige Werte gelten für den Zerstäuber nach der Lehre der vorliegenden Erfindung als besonders bevorzugte Werte.

Fig. 3 zeigt als Beispiel einen schematischen Schnitt durch eine erfindungsgemäße Hochdruckkammer (100), wie sie in einem Zerstäuber beispielsweise gemäß Fig. 1 und 2 eingesetzt sein kann.

Die festigkeitsbestimmenden Komponenten der Hochdruckkammer (100) sind ein im Innern im wesentlichen zylindrisches Zentralteil (123) und die dieses Zentralteil zu beiden Enden abschließenden Bauteile, und zwar der Düsenhalter (133) und der Stützring (126). Diese Bauteile können im montierten Zustand weitere Bauteile wie Dichtungen, Filter und Düse im System einschließen.

Das als spezielle Ausführungsform in Fig. 3 dargestellte Zentralteil (123) umfasst einen metallischen Grundkörper (123a), der im Innern einen im wesentlichen zylindrischen Querschnitt und eine Außenform aufweist, die an die Form der damit verbundenen festigkeitsbestimmenden Bauteile Stützring (126) und Düsenhalter (133) angepasst ist. Diese Verbindungsbereiche können sowohl erhobene als auch vertiefte Strukturen aufweisen, wie z.B. Stege, Vorsprünge, Sicken, lochartige oder rillenförmige Vertiefungen, schlitzartige Aussparungen. In der in Fig. 3 dargestellten Ausführungsform handelt es sich um parallel zur Längsrichtung des Zentralteils (123) verlaufende rechteck- spalt- oder schlitzförmige Aussparungen, in die Verbindungselemente an den Bauteilen (126) und (133) formschlüssig eingreifen. In eine einzige derartige Aussparung können Verbindungselemente von zwei oder mehr Bauteilen eingreifen. In Fig. 3 greift je ein Verbindungselement (126c) des Stützrings (126) in die gleiche Aussparung wie je ein Verbindungselement (133c) des Düsenhalters (133). Jedoch sind andere konstruktive Ausführungen möglich, insbesondere hinsichtlich der Form der Anbindungsstellen, der Anzahl der pro Anbindungsstelle eingreifenden Verbindungselemente und der Anzahl der Bauteile, die über Verbindungselemente an einer Anbindungsstelle eingreifen: Es kann z.B. jeweils nur ein Verbindungselement an eine Anbindungsstelle angebunden sein oder zwei oder mehrere Verbindungselemente. An einem Bauteil können ein oder mehrere andere Bauteile angebunden sein.
Zusätzlich zu den Verbindungselementen mit den festigkeitsbestimmenden Bauteilen der Hochdruckkammer (100) können weitere Verbindungselemente zwecks Einbau der gesamten Hochdruckkammer (100) in einem Gesamtsystem wie einem Zerstäuber direkt an der Außenseite des Zentralteils (123) oder an den Bauteilen (126) oder (133) angeformt sein. Besonders bevorzugt bei diesen weiteren Verbindungselementen sind Federärmchen (123d), die eine Federverrastung oder eine Schnappverbindung mit anderen Bauteilen bewirken.
Das Zentralteil (123) kann ferner einen mit dem metallischen Grundkörper (123a) fest verbundenen Bereich (123b) aufweisen, der aus einem anderen Material, vorzugsweise einem Kunststoff bestehen kann. Dieser zweite Materialbereich (123b) kann alle Oberflächen abdecken, die innerhalb des Gesamtsystems während des Betriebs in Kontakt mit der Flüssigkeit stehen. Bevorzugt wird ein Kunststoff verwendet, der chemisch verträglich mit der eingesetzten Flüssigkeit ist, damit keine Materialkorrosionen und keine Veränderung der Flüssigkeit wie z.B. eine Wirkstoffzersetzung eintritt. Geeignete Kunststoffe sind beispielsweise Polyethylen oder Polypropylen. Derartige Kunststoffe können beispielsweise auch für die im Folgenden beschriebenen flüssigkeitsseitigen zweiten Materialbereiche an anderen Bauteilen wie Stützring (126) und Düsenhalter (133) verwendet werden.
Der zweite Materialbereich - zum Beispiel (123b) - kann eine Beschichtung sein, deren Oberfläche im wesentlichen parallel zur Oberfläche des zugrunde liegenden Grundkörpers - zum Beispiel (123a) - verläuft. Weiter kann der zweite Materialbereich über Anspritzen, Verkleben, Bonden, Verschnappen oder ähnliche Anbindungsmethoden mit dem Grundkörper - zum Beispiel (123a) - verbunden sein. Insbesondere unter Ausnutzen solcher letztgenannten Anbindungsmethoden kann der zweite Materialbereich eine Form haben, die sich von der Form des Grundkörpers unterscheidet. Fig. 3 zeigt ein Zentralteil (123), bei dem sich ein an den Grundkörper (123a) vorzugsweise angespritzter zweiter Materialbereich (123b) mit individuellen Strukturen über gesamten Innenbereich des Zentralteils (123) erstreckt.
Der zweite Materialbereich (123b) dient als Auflagefläche für weitere Bauteile, die beim Zusammenbauen der die Festigkeit der Hochdruckkammer (100) bestimmenden Bauteile Zentralteil (123), Stützring (126) und Düsenhalter (133) eingeschlossenen werden können.

Die Struktur des zweiten Materialsbereichs (123b) gibt die Einbauposition von separaten Filtern, wie einem Vorfilter (27) und einem weiteren Feinfilter (31), vor. Diese Filter können über Presspassung in teilweise konischer Form vom stromaufwärtigen Ende des Zentralteils (123) gegen einen ringförmigen Kragen oder Anschlag in der Innenstruktur des zweiten Materialbereichs (123b) eingeschoben sein. Bei dieser Einbauart sind keine weiteren Bauteile als Filterhalterung oder zur Abdichtung im Bereich der Filter erforderlich. Die Filterschwelle des Feinfilters (31) wird hierbei so gewählt, dass sie zwischen der des Vorfilters (27) und des in der Austragsdüse (12) integrierten Feinstfilters liegt.
Andere konstruktive Ausführungen hinsichtlich des Filtereinbaus, der Filtertypen, der Filteranzahl und des Hintereinanderschaltens von Filtern sind möglich. Die Strukturen des Bereichs (123b) unterbinden jeglichen Kontakt zwischen Flüssigkeit und Grundkörper (123a). Die Dichtungen zu anderen Bauteilen können so am Bereich (123b) anliegen, dass ein direkter Kontakt zwischen Grundkörper (123a) und Dichtung vermieden wird. Die Dichtung (24), z.B. ein O-Rings, dichtet das Zentralteil (123) und den Stützring (126) gegen den Hohlkolben (9) ab. Die Dichtung (24) liegt in einer im Bereich (123b) vorgegebenen Aussparung. Der Bereich (123b) ragt mit einem umlaufenden Verbindungskranz (123c) stromabwärts aus dem Zentralteil (123) heraus und bildet die Auflagefläche für die Düsendichtung (12).

Der in Fig. 3 dargestellte Stützring (126) bildet mit der Dichtung (24) und dem Druckerzeugers (5) die stromaufwärtige Begrenzung der Hochdruckkammer (100). Ebenso wie das Zentralteil (123) kann der Stützring (126) aus unterschiedlichen Materialbereichen aufgebaut sein, die in analoger Weise fest miteinander verbunden sind: einem festigkeitsbestimmenden Grundkörper (126a) und einem direkt angebundenen, mit der Flüssigkeit chemisch verträglichen zweitem Materialbereich (126b). Wie beim Zentralteil (123) ist in der Form des Stützrings (126) die Anbindung zu mindestens einem anderen Bauteil, hier zum Zentralteil (123), vorgegeben: Der Stützring (126) enthält in seinem die Festigkeit bestimmenden Grundkörper (126a) Verbindungselemente (126c), die in Richtung des zu fügenden Bauteils vorspringen und die durch einen Umformprozess formschlüssig mit den entsprechenden Anbindungsformen am Zentralteil (123) verbunden sind. Die vorspringenden Verbindungselemente können unterschiedliche Formen haben: Stege von unterschiedlicher Dicke, Breite und Länge in unterschiedlicher Anzahl, mit umlaufenden Kragen oder rohrartigen Vorsprünge, die ähnlich einer Bördelung umgefaltet sein können oder Zackenkränze, die ähnlich einer Kronkorkencrimpung nach innen oder nach außen an Gegenkonturen anliegen können. Die Verbindungselemente (126c) können bevorzugt zu mehreren (mindestens zwei, bevorzugt vier oder mehr) gleichmäßig über den Umfang des Stützrings (126) verteilt sein. Diese stromabwärts zeigenden Stege oder Ärmchen sind durch einen Umformprozess, wie einen Crimpprozess, in entsprechende Aussparungen am Zentralteil (123) nach innen gebogen und bilden eine formschlüssige und feste Verbindung zwischen den beiden Bauteilen Stützring (126) und Zentralteil (123). Die Form der Crimpverbindung und die Abmessungen der Crimpärmchen sind abhängig vom Material des Grundkörpers (123a) und von den im System abzufangenden Drücken. Mit geeigneten Formen und Materialien für solche Crimpungen können die verbundenen Bauteile einem Innendruck von 5000 bar standhalten. Besonders bevorzugt sind Verbindungselemente (126c) in Form von vier gleichmäßig über den Bauteilrand verteilten quaderförmigen, vorstehenden Ärmchen mit einer Breite zwischen 0,8 und 1,8 Millimeter, einer Länge zwischen 1,6 und 3,0 Millimeter und einer Materialstärke bzw. Dicke zwischen 1,5 und 2,5 Millimeter. Bei einer Veränderung der Anzahl der Ärmchen ist insbesondere deren Breite anzupassen; so wäre bei nur zwei Ärmchen eine Breite zwischen 1,6 und 3,6 Millimeter bevorzugt.
Vom Funktionsprinzip her vergleichbar, aber in den Figuren nicht dargestellt, sind am Zentralteil (123) vorhandene vorspringende Verbindungselemente, die in entsprechende Aussparungen an mindestens einem der weiteren festigkeitsbestimmenden Bauteile ((126) oder (133)) zum Formschluss hin umgeformt sind.

Der zweite Materialbereich (126b) des Stützrings (126) kann analog zum zweiten Materialbereich (123b) des Zentralteils (123) einerseits eine Beschichtung sein, deren Oberfläche im wesentlichen parallel zu der des zugrunde liegenden Grundkörpers (123a) verläuft. Andererseits kann der zweite Materialbereich (126b) durch Anspritzen, Verkleben, Bonden, Verschnappen oder ähnliche Anbindungsmethoden mit dem Grundkörper (126a) verbunden sein und eine angepasste, nach außen hin vom Grundkörper verschiedene Form haben. Fig. 3 zeigt einen Stützring (126), bei dem sich ein an den Grundkörper (126a) angespritzter zweiter Materialbereich (126b) mit nach außen hin vom Grundkörper verschiedener Struktur über den gesamten zum zentralen Hohlkolben (9) hin orientierten Bereich des Stützrings (126) erstreckt. Hierdurch wird einerseits wie im Zentralteil (123) jeglicher direkter Kontakt zwischen Flüssigkeit (2) und metallischem Grundkörper (126a) unterbunden. Andererseits kann der metallische Hohlkolben (9) mit pumpenseitig eingebautem Rückschlagventil (10) zu keinem Zeitpunkt, also auch nicht während der Montage den harten Grundkörper (126a) berühren. Damit wird Beschädigungen während der Montage vorgebeugt. Der zweite Materialbereich (126b) kann darüber hinaus mit Einführschrägen versehen sein und/oder spezielle Gleiteigenschaften haben, die das Montieren des Hohlkolbens erleichtern.
Der zweite Materialbereich (126b) ist weiter Auflagefläche der Dichtung (24) der Hochdruckkammer (100). Die Dichtung (24) wird beim Montieren der Bauteile Zentralteil (123) und Stützring (126) im System eingeschlossenen. Der zweite Materialbereich (126b) ragt in das eine Aufnahmebuchse für die Dichtung (24) bildende Zentralteil (123) hinein. Die Dichtung (24) hat so keinen direkten Kontakt zum Grundkörper (126a). Die gegebenenfalls durch das Dichtungsmaterial hindurch gehende Flüssigkeit innerhalb der Hochdruckkammer (100) hat keinen Kontakt mit dem Material des Grundkörpers (126a).

Bei Bedarf können neben Filtern ((27) und (31)) oder Dichtung (24) weitere Bauteile durch das Montieren von Zentralteil (123) und Stützring (126) eingeschlossen sein, z.B. ein Abstandshalter (36) in Form einer flachen Unterlegscheibe mit zentrischer Bohrung. Mit der Dicke des Abstandshalters (36) kann die Eindringtiefe des zweiten Materialbereichs am Stützring (126b) in das Zentralteil (123) verändert werden. Damit kann der Verpressungsgrad und die Vorspannung der Dichtung (24) eingestellt werden.

Bei einer weiteren - nicht in den Figuren dargestellten - Ausführungsform der Hochdruckkammer kann die Dichtfunktion eines Dichtelements - wie einer der Dichtungen (24) oder (29) oder die Düsendichtung (30) - von einem zweiten Materialbereich an einem der festigkeitsbestimmenden Bauteile Zentralteil (123), Stützring (126) oder Düsenhalter (133) übernommen werden. Der zweite Materialbereich kann aus einem handelsüblichen Elastomer - wie Silikon - bestehen und analog zu den oben anhand von Zentralteil (123) und Stützring (126) beschriebenen Materialverbunden mit dem jeweiligen Grundkörper verbunden sein.
Bei einer weiteren - nicht in den Figuren dargestellten - Ausführungsform der Hochdruckkammer kann die Anzahl der festigkeitsbestimmenden Bauteile von drei auf zwei herabgesetzt sein. Dafür kann das Zentralteils (123) entweder mit dem Stützring (126) oder dem Düsenhalter (133) zu einem einzigen Bauteil zusammengefasst sein. Dies kann über das Anspritzung von Dichtungen erreicht werden. Wird die Funktion der Dichtung (24) von dem zweiten Materialbereich (123b) - z.B. bestehend aus einem Elastomer - des Zentralteils übernommen, entfällt die in Fig. 3 vom Stützring (126) gebildete Gegenhalterung dieser Dichtung.

Der in Fig. 3 dargestellte Düsenhalter (133) bildet mit der Düsendichtung (30) und der Austragsdüse (12) die stromabwärtige Begrenzung der Hochdruckkammer (100). Wie das Zentralteil (123) und der Stützring (126) umfasst der Düsenhalter (133) zwei Materialbereiche: einen festigkeitsbestimmenden Grundkörper (133a) und einen direkt angebundenen zweiten Materialbereich (133b), der mit der Flüssigkeit (2) chemisch verträglich ist. Wie beim Zentralteil (123) und beim Stützring (126) ist durch die Form des Düsenhalters (133) die Anbindung zum Zentralteil (123) vorgegeben: Der Düsenhalter (133) umfasst am Grundkörper (133a) in Richtung des zu fügenden Bauteils vorspringende Verbindungselemente (133c), die durch Umformen formschlüssig mit den entsprechenden Formen am Zentralteil (123) verbunden sind. Die vorspringenden Verbindungselemente können aus einer Vielzahl von geometrischen Formen bestehen: Stege unterschiedlicher Breite, Dicke, Länge und Anzahl, umlaufende Kragen oder rohrartige Vorsprünge, die ähnlich einer Bördelung umgefaltet werden können, Zackenkränze, die ähnlich einer Kronkorkencrimpung nach innen an Gegenkonturen anliegen. Die Verbindungselemente (133c) können mehrere (mindestens zwei, bevorzugt vier oder mehr) über den Umfang des Düsenhalters (133) gleichmäßig verteilte, stromaufwärts zeigende Stege oder Ärmchen sein. Beim Umformen - z.B. im Rahmen eines Crimpprozesses - werden die Verbindungselemente in entsprechende Aussparungen am Zentralteil (123) gedrückt, Dadurch entsteht eine formschlüssige und feste Verbindung zwischen den beiden Bauteilen. Die Auslegung der Verbindungselemente (133c) entspricht denen am Stützring (126): Besonders bevorzugt sind Verbindungselemente (133c) in Form von vier gleichmäßig über den Bauteilrand verteilten quaderförmigen, vorstehenden Ärmchen mit einer Breite zwischen 0,8 und 1,8 Millimeter, einer Länge zwischen 1,6 und 3,0 Millimeter und einer Materialstärke bzw. Dicke zwischen 1,5 und 2,5 Millimeter. Bei einer Veränderung der Anzahl der Ärmchen ist insbesondere deren Breite anzupassen; so wäre bei nur zwei Ärmchen eine Breite zwischen 1,6 und 3,6 Millimeter bevorzugt.

Der zweite Materialbereich (133b) des Düsenhalters (133) kann analog zum zweiten Materialbereich (123b) des Zentralteils (123) und zum zweiten Materialbereich (126b) des Stützrings (126) einerseits als Beschichtung ausgeführt sein, bei der die Oberfläche des zweiten Materialbereichs (133b) im wesentlichen parallel zu der des zugrunde liegenden Grundkörpers (133a) verläuft. Andererseits kann der zweite Materialbereich durch Anspritzen, Verkleben, Bonden, Verschnappen oder andere Verfahren mit dem Grundkörper (133a) verbunden sein.

Für die Herstellung der Grundkörper der festigkeitsbestimmenden Bauteile der Hochdruckkammer (100) - Düsenhalter (133), Zentralteil (123) und Stützring (126) - wird die Anwendung eines Metall-Spritzgussprozesses bevorzugt.

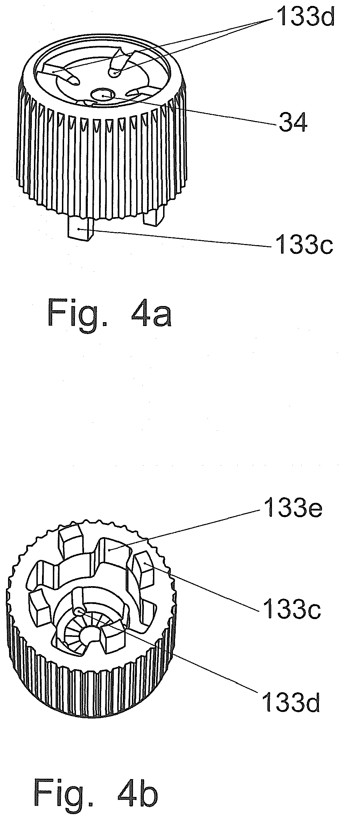

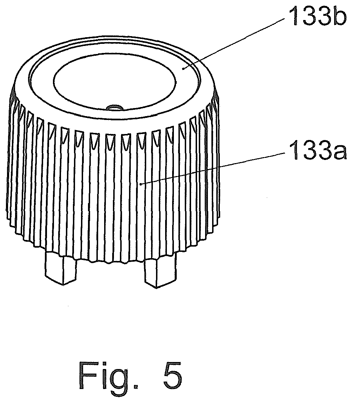

Fig. 4 und Fig. 5 zeigen einen Düsenhalter (133), der in einer Hochdruckkammer (100) entsprechend Fig. 3 eingesetzt werden kann, in verschiedenen Fertigungsstadien: Der im MIM-Verfahren gefertigte Grundkörper (133a) ist in Fig. 4a und Fig. 4b in zwei Perspektiven dargestellt. Die Form des Grundkörpers (133a) ist an die anschließenden Verfahrens- und Montageschritte angepasst: Anspritzen eines zweiten Materialbereichs, Zusammenfügen mit weiteren Bauteilen und deren Anbinden durch einen Crimpprozess.
Für das Anspritzen des zweiten Materialbereichs aus Kunststoff sind im Grundkörper (133a) Fließkanäle (133d) vorgesehen. Der Grundkörper (133a) wird als Einlegeteil in die Form einer Spritzgussmaschine gelegt. Die verflüssigte Spritzgussmasse verteilt sich mit Hilfe der Fließkanäle gleichmäßig auf den für sie vorgesehenen Bereichen des Grundkörpers. Parallel zur Achse der Durchgangsbohrung (34) verlaufende Fließkanäle (133d) ermöglichen das gleichzeitige Anspritzen von Material an gegenüberliegenden Oberflächen des Grundkörpers (133a). Die Durchgangsbohrung (34) gibt nach Montage die Auslassöffnung der Austragsdüse (12) frei. Der zweite Materialbereich (133b) ist so gestaltet, dass jeglicher Kontakt zwischen Flüssigkeit (2) und dem metallischen Grundkörper (133a) unterbunden wird. Dies kann sich nicht nur auf die noch in der Hochdruckkammer (100) befindliche Flüssigkeit (2), sondern auch auf das aus der Düse austretende Aerosol (4) beziehen, dessen Wolke den Düsenhalter (133) im oberen Bereich benetzen kann.

Unter Ausnutzung der dem Fachmann bekannten Möglichkeiten der Metall- und Kunststoff-Spritzgusstechniken sind weitere - hier nicht dargestellte - Ausführungsformen möglich, bei denen die Aerosol-seitige Oberfläche des Düsenhalters (133) zum Durchgangsloch (34) von weiteren nach oben und zur Halterseite hin offenen Kanälen durchzogen sein kann. Wird beim Einatmen des Patienten Seitenluft durch die Zuluftöffnungen (15) in das Innere des Mundstücks (13) angesaugt, kann diese Seitenluft über die neuen Kanäle an den Entstehungsort des Aerosols (14) herangeführt werden und dort als Hüllströmung wirken. Auf diese Weise kann die Ausrichtung der Aerosolwolke weiter unterstützt sowie die Abscheidung von Aerosolpartikeln auf dem Düsenhalter (133) oder im Innenbereich des Mundstücks (13) herabgesetzt werden.

Falls gewünscht, kann ein orientiertes Zusammenfügen des Düsenhalters (133) beim Anlegen an das Zentralteil (123) über eine Positionierhilfe am Düsenhalter (133e) vorgegeben werden. Diese Positionierhilfe am Düsenhalter (133e) kann über an der Fügestelle befindlichen Konturen in beliebiger Form gestaltet sein, so lange diese ein Negativ zu der entsprechenden Kontur des zu fügenden Bauteil ist. Wird ein orientierungsfreies Zusammenfügen angestrebt, so kann die Kontur an der Fügestelle z.B. die Form einer azimutalen Rille annehmen, in welche die entsprechende Crimpung eingreift.
Zusätzlich kann die Mantelfläche des Grundkörpers (133a) und damit des Düsenhalters (133) Griffflächen und Einführschrägen für die folgenden Prozessschritte aufweisen. Eine solche Grifffläche ist in Fig. 4 und Fig. 5 durch eine umlaufende Riffelung des Grundkörpers (133a) dargestellt. In Fig. 4a und Fig. 4b sind die Verbindungselemente (133c) in Form von vier Crimpärmchen dargestellt. Diese geradlinig verlaufenden Crimpärmchen werden im späteren Anbindungs-Prozess nach innen hin gebogen und liegen formschlüssig in entsprechenden Aussparungen am gefügten Zentralteil (123) an. Im Ausführungsbeispiel gemäß Fig. 3 werden die Bauteile Austragsdüse (12) und Düsendichtung (30) durch Fügen und Verbinden von Düsenhalter (133) und Zentralteil (123) in der Hochdruckkammer (100) eingeschlossen. Die Austragsdüse (12) bildet den stromabwärtigen Abschluss der Hochdruckkammer (100). Damit das Zentralteil (123) gegen den Düsenhalter (133) abgedichtet ist und die Flüssigkeit den Grundkörper (133a) des Düsenhalters (133) nicht berührt, liegt die Austragsdüse (12) analog zu den von Zentralteil (123) und Stützring (126) eingeschlossenen Bauteilen nur am zweiten Materialbereich (133b) des Düsenhalters (133) an. Dies hat im Falle von spröden Materialien des Düsenkörpers oder empfindlichen Mikrostrukturen den Vorteil, dass der Düsenkörper beim Einfügen auf eine im Vergleich zum steifen Grundkörper (133a) weichere Oberfläche trifft und so Beschädigungen des Düsenkörpers z.B. beim Einbau vermieden werden.

Im folgenden werden noch einmal der bevorzugte Fertigungsprozess für die die Festigkeit der Hochdruckkammer (100) bestimmenden Bauteile Zentralteil (123) und Stützring (126) und Düsenhalter (133) und das Fügen dieser Bauteile schematisch zusammengefasst:
- Bereitstellen einer Granulat-Mischung aus einem Metallpulver und aus einem Binder
- Einspritzen der geschmolzenen Granulat-Mischung in eine Form in einer Spritzgießmaschine
- Entformen des Rohlings
- Entbindern des Rohlings (z.B. durch Wärmebehandlung)
- Sintern des Rohlings und Gewinnung des jeweiligen Grundkörpers (123a), (126a) oder (133a) aus Sintermetall
- Bereitstellen eines Kunststoff-Granulats
- Einlegen eines Grundkörpers in eine Form in einer Spritzgießmaschine
- Einspritzen des geschmolzenen Kunststoff in die Form in der Spritzgießmaschine
- Entformen des Verbundbauteils (123), (126) oder (133)
- Gegebenenfalls Einlegen von Bauteile in ein Verbundbauteil (z.B. Düsendichtung (30) mit Austragsdüse (12) in den Düsenhalter (133); Feinfilter (31), Vorfilter (27) und Dichtung (24) in das Zentralteil (123))
- Fügen der die Festigkeit der Hochdruckkammer bestimmenden Bauteile miteinander
- Verbinden der gefügten Bauteile durch Umformen der Verbindungselemente, insbesondere durch Crimpen von Crimpärmchen

Zur Materialwahl für Bauteile aus einem MIM-Prozess (insbesondere für die Bauteile mit umzuformenden Verbindungselementen wie Stützring (126a) oder Düsenhalter (133a) aus
Fig. 3) werden bevorzugt solche Metalle ausgewählt, bei denen sich durch das Umformen bzw. Crimpen das Material verfestigt und die Druckfestigkeit des Hochdrucksystems erhöht wird. Eines von vielen Beispielen für ein solches Metall ist der Edelstahl US-AISA 317F, wie er bei der Fa. Strack, Deutschland, erhältlich ist. Dieses Metall lässt sich ohne zusätzlichen Wärmeeintrag von außen kalt crimpen.
Crimpverbindungen sind unlösbare Verbindungen, d.h. sie lassen sich ohne Beschädigung der Bauteile nicht lösen. Eine Crimpverbindung kann zwar durch vorsichtiges Zurückbiegen der Crimpärmchen geöffnet werden, nur können u.a. aufgrund der Materialverfestigung beim ersten Crimpen die Crimpärmchen nicht mehr in ihren ursprünglichen Ausgangszustand zurückversetzt werden, falls sie sich überhaupt ohne Bruch zurück biegen lassen. Beim Öffnen einer Crimpverbindung ist zumindest mit Materialabnutzungen oder Materialveränderungen zu rechnen.

Dieser Fertigungsprozess kann je nach Forderungen, die an die Bauteile gestellt werden, sowie je nach Komplexität der Bauteilen und Systeme fast beliebig nach grundsätzlich dem Fachmann bekannten Methoden verändert werden. So können zusätzliche Forderungen an ein Bauteil oder das System über einen dritten Materialbereich an ausgewählten Bauteilen erfüllt werden, wenn ein weiterer Satz an Verfahrensschritten zum Beschichten oder Umspritzen von Bauteilen eingefügt wird.
Die festigkeitsbestimmenden Bauteile der Hochdruckpumpe können aus unterschiedlichen Prozessen stammen. Beispielsweise kann das Zentralteil (23) ganz nach Forderungen und Materialpreisen direkt aus einem hochwertigen Kunststoff wie vorzugsweise dem vergleichsweise teuren PEEK (Polyether-Ether-Keton) bestehen.
Die festigkeitsbestimmenden Bauteile können aus anderem Material als Metall oder Sintermetall bestehen. Das Zentralteil (123) kann anstelle der Herstellung des Grundkörpers im MIM-Verfahren aus einem gezogenen Rohr bestehen, das die Festigkeit vorgibt. Das Rohr kann als Einlegeteil in eine Spritzgießmaschine eingesetzt werden. Die innen und außen liegenden Strukturen könen in einem Kunststoffspritzgießprozess an das Rohr angeformt werden.

Die vorliegende Erfindung hat folgende Vorteile:
- Wirtschaftliche Massenfertigung von präzisen Bauteilen aus Sintermetall
- Vielfältige, komplizierte Formen der Bauteile sind fast ohne zusätzliche Produktionskosten erhältlich
- In der Form der Bauteile enthaltene Verbindungselemente und Positionierhilfen ermöglichen die schnelle und wirtschaftliche Montage der Bauteile
- Durch Crimpen verbundene Bauteile sind für einen Flüssigkeitsdruck bis zu 5000 bar geeignet.
- Crimpverbindungen sind bezüglich der Lagetoleranzen weniger fehleranfällig als Schraubverbindungen.
- Die Form der Bauteile aus Sintermetall ist nicht auf rotationssymmetrische Formen beschränkt.
- Als Sintermetall können Metalle eingesetzt werden, die bei bekannten Verfahren nur schwer verarbeitbar sind.
- Erfüllung verschiedenster Forderungen an das Material eines Bauteils (Steifigkeit, Robustheit, Materialverträglichkeit, Korrosionsbeständigkeit) durch 2-Komponenten-Spritzguss
- Kompakte Bauweise der Hochdruckkammer, geeignet für Einsatz in Handgeräten
- Geringes Totvolumen durch Innenbeschichtung und Minimierung der Komponenten der Hochdruckkammer
- Vermeiden von Beschädigungen beim Montieren von Bauteilen im Innern der Hochdruckkammer durch Beschichten mit Kunststoff

Wird die erfindungsgemäße Hochdruckkammer im medizinischen Bereich eingesetzt, ist die Flüssigkeit (2) vorzugsweise eine Arzneimittelzubereitung.
Nachfolgend werden bevorzugte Verbindungen bzw. pharmazeutische Wirkstoffe, Bestandteile und/oder Formulierungen von medizinischen Flüssigkeiten aufgeführt. Dabei kann es sich um beliebige Mischungen mit vielfältigen anderen Flüssigkeiten handeln. Pulverförmige Substanzen können sowohl in Wasser als auch in einem beliebigen Lösemittel gelöst werden oder als Suspension vorliegen.

Die unten genannten pharmazeutischen Verbindungen können allein oder in Kombination zur Anwendung in der erfindungsgemäßen Vorrichtung gelangen. In den unten genannten Verbindungen ist W ein pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quino lin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quino lin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC**-**1** 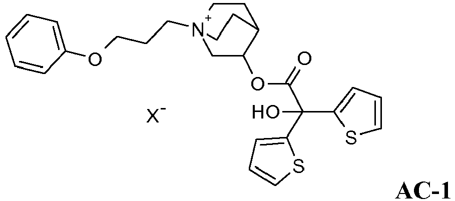 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 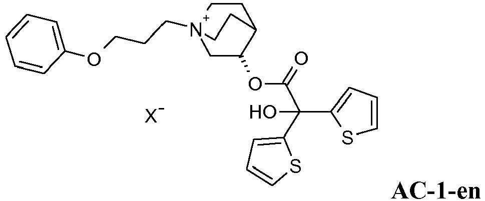 enthalten, worin X⁻ die vorstehend genannten Bedeutungen aufweisen kann. Weiterhin bevorzugte Anticholinergika sind ausgewählt aus den Salzen der Formel **AC-2** 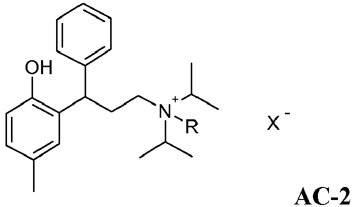 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

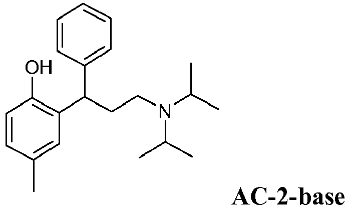

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.
Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason,

Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6□,9□-difluoro-11□-hydroxy-16□-methyl-3-oxo-17□-(2,2,3,3-tertamethylcyclopropylcarbonyl)oxy-androsta-1,4-diene-17□-carbonsäure cyanomethyl ester gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl] amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl] amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl] amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazo lin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazo lin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexen-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexen-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazo lin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazo lin
- 4-[(3-Chlor-4-fluor-phenyl)amino]-6-[1-(2-methoxy-acetyl)-piperidin-4-yloxy]-7-(2-methoxy-ethoxy)-chinazo lin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazo lin
- 4-[(3-Chlor-4-fluor-phenyl)amino]-6-(cis-4-methylamino-cyclohexan-1-yloxy)-7-methoxy-chinazo lin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazo lin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexen-1-yloxy]-7-methoxy-chinazolin
- 4-[(3-Chlor-4-fluor-phenyl)amino]-6-[cis-4-(N-acetyl-N-methyl-amino)-cyclohexan-1 - yloxy]-7-methoxy-chinazolin
- 4-[(3-Chlor-4-fluor-phenyl)amino]-6-(trans-4-methylamino-cyclohexan-1-yloxy)-7-methoxy-chinazo lin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1 | Zerstäuber | 27 | Vorfilter |
| 2 | Flüssigkeit | 28 | Filterhalter |
| 3 | Behälter | 29 | Dichtung |
| 4 | Beutel | 30 | Düsendichtung |
| 5 | Druckerzeuger | 31 | Feinfilter |
| 6 | Halterung | 32 | Düsenhalter |
| 7 | Antriebsfeder | 33 | Überwurfmutter |
| 8 | Sperrelement | 34 | Durchgangsloch |
| 9 | Hohlkolben | 36 | Abstandshalter |
| 10 | Rückschlagventil | | |
| 11 | Druckkammer | 100 | Hochdruckkammer |
| 12 | Austragsdüse | 123 | Zentralteil |
| 12a | Platte | 123a | Grundkörper |
| 12b | Platte | 123b | zweiter Materialbereich |
| 13 | Mundstück | 123c | Verbindungskranz |
| 14 | Aerosol | 123d | Federärmchen |
| 15 | Zuluftöffnung | 126 | Stützring |
| 16 | Gehäuseoberteil | 126a | Grundkörper |
| 17 | Gehäuseinnenteil | 126b | zweiter Materialbereich |
| 18 | Gehäuseunterteil | 126c | Verbindungselement |
| 19 | Halteelement | 133 | Düsenhalter |
| 20 | Feder (im Gehäuseunterteil) | 133a | Grundkörper |
| 21 | Behälterboden | 133b | zweiter Materialbereich |
| 22 | Anstechelement | 133c | Verbindungselement |
| 23 | Zentralteil | 133d | Fließkanal |
| 24 | Dichtung | 133e | Positionierhilfe am |
| 25 | Stützring | | Düsenhalter |
| 26 | Überwurfmutter | | |

Anmerkung: Für die Ziffern 37 - 99 sind keine Bezugszeichen in den Figuren vergeben

## Patentansprüche

1. Hochdruckkammer in Form einer mehrteiligen Pumpenkammer mit Austragsdüse (12), wobei die Hochdruckkammer geeignet ist, um darin eine Flüssigkeit (2) unter Drücke von 100 bis 1200 bar zu setzen, und wobei die Hochdruckkammer mindestens ein Metallbauteil aus einem umformbaren und/oder crimpfähigen Metall aufweist und dieses mindestens eine Metallbauteil durch Umformen oder Crimpen mit mindestens einem anderen Bauteil der Hochdruckkammer formschlüssig unlösbar verbunden ist, **dadurch gekennzeichnet, dass** an dem mindestens einen Metallbauteil ein zweiter Materialbereich (123b, 126b, 133b) aus Kunststoff in einem Kunststoff-Spritzguss-Prozess direkt angebunden worden ist oder dass das mindestens eine Metallbauteil zumindest teilweise mit einem zweiten Material aus Kunststoff ausgekleidet oder beschichtet ist.

2. Hochdruckkammer nach Anspruch 1 **dadurch gekennzeichnet, dass** das zweite Material derart gewählt ist, dass es korrosionsbeständig und/oder pharmazeutisch verträglich gegenüber der für den Einsatz vorgesehenen Flüssigkeit (2) ist.

3. Hochdruckkammer nach einem der Ansprüche 1 oder 2 **dadurch gekennzeichnet, dass** der zweite Materialbereich (123b, 126b, 133b) oder eine Beschichtung oder Auskleidung alle Oberflächen des mindestens einen Metallbauteils bedeckt, die beim Betrieb der Hochdruckkammer direktem Kontakt mit der Flüssigkeit (2) haben.

4. Hochdruckkammer nach einem der Ansprüche 1 oder 3 **dadurch gekennzeichnet, dass** der Kunststoff Polypropylen oder Polyethylen ist.

5. Hochdruckkammer nach einem der Ansprüche 1 oder 2 **dadurch gekennzeichnet, dass** das mindestens eine Metallbauteil ein festigkeitsbestimmendes Bauteil ist und der zweite Materialbereich (123b, 126b, 133b) aufgrund seiner Form und seiner Materialbeschaffenheit an der Verbindungsstelle mit einem weiteren festigkeitsbestimmenden Bauteil eine Dichtung (24, 29) des Innenraums der Hochdruckkammer darstellt.

6. Hochdruckkammer nach Anspruch 1 **dadurch gekennzeichnet, dass** die festigkeitsbestimmenden Bauteile der Hochdruckkammer einen Düsenhalter (133) und/oder ein Zentralteil (123) und einen kolbenseitigen Stützring (126) umfassen.

7. Hochdruckkammer nach Anspruch 1 **dadurch gekennzeichnet, dass** das crimpfähige Metall zusätzlich die Eigenschaft aufweist, dass durch mechanisches Umformen eine Materialverfestigung auftritt.

8. Hochdruckkammer nach einem der Ansprüche 1, 6 oder 7 **dadurch gekennzeichnet, dass** durch Umformen oder Crimpen separate Einzelbauteile in der Hochdruckkammer eingeschlossen sind, die dichtende und/oder filternde und/oder Flüssigkeitszerstäubende und/oder einstellende Funktionen haben.

9. Hochdruckkammer nach einem der Ansprüche 1, 6 oder 8 **dadurch gekennzeichnet, dass** die Art der Verbindung des mindestens einen mit einem anderen Bauteil zu verbindenden Metallbauteils oder die Gestalt der Crimpung bereits durch die Form von Verbindungselementen (126c, 133c, 123d) an dem mindestens einen Metallbauteil vorgegeben sind.

10. Hochdruckkammer nach Anspruch 9 **dadurch gekennzeichnet, dass** die Verbindungselemente (126c, 133c) an dem mindestens einen Metallbauteil die Form von mindestens zwei vorzugsweise vier zum anderen Bauteil hin geknickte Stege oder Ärmchen haben und dass das mindestens eine Metallbauteil über Crimpen mit dem anderen Bauteil verbundenen ist.

11. Hochdruckkammer nach Anspruch 10 **dadurch gekennzeichnet, dass** die beim Crimpen geknickten Stege oder Ärmehen (126c, 133c) eine Breite zwischen 0,8 und 3,6 Millimeter und/oder eine Länge zwischen 1,6 und 3,0 Millimeter und eine Materialstärke zwischen 1,5 und 2,5 Millimeter haben.

12. Hochdruckkammer nach Anspruch 6 **dadurch gekennzeichnet, dass** der Düsenhalter (32, 133) auf der Seite des Auslasses der Austragsdüse (12) von mindestens einem vorzugsweise nach oben offenen Kanal durchzogen ist.

13. Hochdruckkammer nach Anspruch 1 **dadurch gekennzeichnet, dass** das Innenvolumen der Kammer bis 250 Mikroliter vorzugsweise 10 bis 100 Mikroliter beträgt und/oder die Hochdruckkammer auf einen Überdruckbereich bis zu 1200 bar ausgelegt ist.

14. Zerstäuber (1) oder Injektor zur Zerstäubung oder Injektion einer Flüssigkeit (2), insbesondere einer Arzneimittelformulierung, mit einer Hochdruckkammer gemäß einem der Ansprüche 1-8.

15. Verfahren zur Herstellung einer Hochdruckkammer nach Anspruch 1 umfassend als Bauteile einen Düsenhalter (32, 133) und/oder ein Zentralteil (23, 123) und einen Stützring (25, 126) **dadurch gekennzeichnet, dass** mindestens zwei der Bauteile in einem Verfahrensschritt ineinander gesteckt und durch Umformen oder Crimpen miteinander verbunden werden, wobei
mindestens eines der Bauteile Düsenhalter (133), Zentralteil (123) oder Stützring (126) einen Grundkörper (133a, 123a, 126a) aufweist, der vor dem Crimpen in einem separaten Spritzgussprozess mit einem zweiten Materialbereich (123b, 126b, 133b), einer Beschichtung oder Auskleidung versehen wird.

16. Verfahren nach Anspruch 15 **dadurch gekennzeichnet, dass** der Grundkörper (133a, 123a, 126a) des mindestens einen Bauteils Düsenhalter (133), Zentralteil (123) oder Stützring (126) in einem Metall-Spritzguss-Verfahren gefertigt wird.

## Claims

1. High-pressure chamber in the form of a multipart pump chamber which comprises a discharge nozzle (12), wherein the high-pressure chamber is capable of applying pressures of from 100 to 1,200 bar to a liquid (2) therein, and wherein the high-pressure chamber has at least one metal component made of a metal that can be deformed and/or crimped, said at least one metal component being non-detachably connected in an interlocking manner to at least one other component of the high-pressure chamber by means of deformation or crimping, **characterised in that** a second material region (123b, 126b, 133b) made of plastics material has been directly attached to the at least one metal component in a plastics injection moulding process, or **in that** the at least one metal component is lined or coated, at least in part, with a second material made of plastics material.

2. High-pressure chamber according to claim 1, **characterised in that** the second material is selected so as to be corrosion-resistant to and/or pharmaceutically compatible with the liquid (2) provided for use.

3. High-pressure chamber according to either claim 1 or claim 2, **characterised in that** the second material region (123b, 126b, 133b) or a coating or lining covers all surfaces of the at least one metal component that come into direct contact with the liquid (2) during operation of the high-pressure chamber.

4. High-pressure chamber according to either claim 1 or claim 3, **characterised in that** the plastics material is polypropylene or polyethylene.

5. High-pressure chamber according to either claim 1 or claim 2, **characterised in that** the at least one metal component is a strength-defining component and the second material region (123b, 126b, 133b) provides a seal (24, 29) for the interior space of the high-pressure chamber at the connection point with an additional strength-defining component due to the shape of said second material region and the material properties thereof.

6. High-pressure chamber according to claim 1, **characterised in that** the strength-defining components of the high-pressure chamber include a nozzle holder (133) and/or a central part (123) and a piston-side bearing ring (126).

7. High-pressure chamber according to claim 1, **characterised in that** the metal that is able to be crimped additionally has the feature whereby material hardening occurs by means of mechanical deformation.

8. High-pressure chamber according to any of claims 1, 6 or 7, **characterised in that** separate individual components, which have sealing and/or filtering and/or liquid atomising and/or adjusting functions, are enclosed in the high-pressure chamber by means of deformation or crimping.

9. High-pressure chamber according to any of claims 1, 6 or 8, **characterised in that** the type of connection of the at least one metal component to be connected to another component or the shape of the crimping is predetermined by the form of the connection elements (126c, 133c, 123d) on the at least one metal component.

10. High-pressure chamber according to claim 9, **characterised in that** the connection elements (126c, 133c) on the at least one metal component are in the form of at least two, preferably four, projections or arms that are bent towards the other component, and **in that** the at least one metal component is connected to the other component by means of crimping.

11. High-pressure chamber according to claim 10, **characterised in that** the projections or arms (126c, 133c), which are bent during crimping, have a width of between 0.8 and 3.6 millimetres and/or a length of between 1.6 and 3.0 millimetres and a material thickness of between 1.5 and 2.5 millimetres.

12. High-pressure chamber according to claim 6, **characterised in that** at least one channel passes through the nozzle holder (32, 133) on the side of the outlet of the discharge nozzle (12), which channel is preferably open at the top.

13. High-pressure chamber according to claim 1, **characterised in that** the internal volume of the chamber is up to 250 microlitres, preferably from 10 to 100 microlitres, and/or the high-pressure chamber is designed to have an overpressure range of up to 1,200 bar.

14. Atomiser (1) or injector for atomising or injecting a liquid (2), in particular a pharmaceutical formulation, comprising a high-pressure chamber according to any of claims 1 to 8.

15. Method for producing a high-pressure chamber according to claim 1 comprising a nozzle holder (32, 133) and/or a central part (23, 123) and a bearing ring (25, 126) as components, **characterised in that** at least two of the components are pushed into one another in a method step and are interconnected by means of deformation or crimping, wherein at least one component from the nozzle holder (133), central part (123) or bearing ring (126) comprising a main body (133a, 123a, 126a) is provided with a second material region (123b, 126b, 133b), coating or lining in a separate injection moulding process before crimping.

16. Method according to claim 15, **characterised in that** the main body (133a, 123a, 126a) of the at least one component from the nozzle holder (133), central part (123) or bearing ring (126) is made by means of a metal injection moulding method.

## Revendications

1. Chambre à haute pression en forme d'une chambre de pompe en plusieurs parties avec buse de décharge (12), dans laquelle la chambre à haute pression est adaptée pour mettre dedans un liquide (2) sous une pression de 100 à 1200 bar, et dans laquelle la chambre à haute pression présente au moins un élément métallique en un métal formable et/ou sertissable et ledit au moins un élément métallique est relié de manière imperdable par assemblage de forme par formage ou sertissage à au moins un autre élément de la chambre à haute pression, **caractérisée en ce qu'**une seconde zone de matériau (123b, 126b, 133b) en plastique est reliée directement à l'au moins un élément métallique lors d'un processus de moulage par injection de plastique ou **en ce qu'**au moins un élément métallique est recouvert ou revêtu au moins en partie d'un second matériau en plastique.

2. Chambre à haute pression selon la revendication 1, **caractérisée en ce que** le second matériau est sélectionné de telle sorte qu'il est résistant à la corrosion et/ou pharmaceutiquement acceptable vis-à-vis du liquide (2) dont l'utilisation est prévue.

3. Chambre à haute pression selon l'une des revendications 1 ou 2, **caractérisée en ce que** la seconde zone de matériau (123b, 126b, 133b) ou un revêtement ou un recouvrement recouvre toutes les surfaces de l'au moins un élément métallique qui, lors du fonctionnement de la chambre à haute pression, sont en contact direct avec le liquide (2).

4. Chambre à haute pression selon l'une des revendications 1 ou 3, **caractérisée en ce que** le plastique est du polypropylène ou du polyéthylène.

5. Chambre à haute pression selon l'une des revendications 1 ou 2, **caractérisée en ce que** l'au moins un élément métallique est un élément déterminant la résistance et la seconde zone de matériau (123b, 126b, 133b), du fait de sa forme et de la nature de son matériau, constitue, au niveau de la jonction avec un autre élément déterminant la résistance, un joint d'étanchéité (24, 29) de l'intérieur de la chambre à haute pression.

6. Chambre à haute pression selon la revendication 1, **caractérisée en ce que** les éléments déterminant la résistance de la chambre à haute pression comprennent un support de buse (133) et/ou une pièce centrale (123) et une bague d'appui côté piston (126).

7. Chambre à haute pression selon la revendication 1, **caractérisée en ce que** le métal sertissable présente en outre la propriété que le formage mécanique donne lieu à une solidification du matériau.

8. Chambre à haute pression selon l'une des revendications 1, 6 ou 7, **caractérisée en ce que** des éléments individuels séparés sont inclus dans la chambre à haute pression par formage ou sertissage, qui ont des fonctions d'étanchéification et/ou de filtrage et/ou de pulvérisation de liquide et/ou de réglage.

9. Chambre à haute pression selon l'une des revendications 1, 6 ou 8, **caractérisée en ce que** la nature de la liaison de l'au moins un élément métallique devant être relié à un autre élément ou la forme du sertissage sont déjà définis par la forme d'éléments de liaison (126c, 133c, 123d) au niveau de l'au moins un élément métallique.

10. Chambre à haute pression selon la revendication 9, **caractérisée en ce que** les éléments de liaison (126c, 133c) présentent, au niveau de l'au moins un élément métallique, la forme d'au moins deux, de préférence quatre ailettes ou petits bras pliés vers l'autre élément et **en ce que** l'au moins un élément métallique est relié par sertissage à l'autre élément.

11. Chambre à haute pression selon la revendication 10, **caractérisée en ce que** les ailettes ou petits bras pliés lors du sertissage (126c, 133c) ont une largeur comprise entre 0,8 et 3,6 millimètres et/ou une longueur comprise entre 1,6 et 3,0 millimètres et une épaisseur de matériau comprise entre 1,5 et 2,5 millimètres.

12. Chambre à haute pression selon la revendication 6, **caractérisée en ce que** le support de buse (32, 133) est parcouru sur le côté de la sortie de la buse de décharge (12) par au moins un canal ouvert de préférence vers le haut.

13. Chambre à haute pression selon la revendication 1, **caractérisée en ce que** le volume intérieur de la chambre est de jusqu'à 250 microlitres, de préférence de 10 à 100 microlitres et/ou la chambre à haute pression est conçue pour une zone de surpression allant jusqu'à 1200 bar.

14. Pulvérisateur (1) ou injecteur pour la pulvérisation ou l'injection d'un liquide (2), en particulier d'une formulation de médicament, avec une chambre à haute pression selon l'une des revendications 1 à 8.

15. Procédé de fabrication d'une chambre à haute pression selon la revendication 1, comprenant comme éléments un support de buse (32, 133) et/ou une partie centrale (23, 123) et une bague d'appui (25, 126), **caractérisé en ce qu'**au moins deux des éléments sont emboîtés l'un dans l'autre lors d'une étape de procédé et reliés l'un à l'autre par formage ou sertissage, dans lequel
au moins un des éléments support de buse (133), partie centrale (123) ou bague d'appui (126) présente un corps de base (133a, 123a, 126a) qui, avant le sertissage, est muni d'une seconde zone de matériau (123b, 126b, 133b), d'un revêtement ou d'un recouvrement lors d'un processus de moulage par injection séparé.

16. Procédé selon la revendication 15, **caractérisé en ce que** le corps de base (133a, 123a, 126a) de l'au moins un élément support de buse (133), pièce centrale (123) ou bague d'appui (126) est fabriqué dans un procédé de moulage par injection de métal.
